# EUROPEAN PATENT SPECIFICATION

(11) **EP 1 581 159 B1**
(45) Date of publication and mention of the grant of the patent: **11.04.2012**
(21) Application number: 04701142.4
(22) Date of filing: 09.01.2004
(51) Int. Cl.: A61F 13/15

(54) **METHOD FOR PRODUCING DIAPER PANTS OF THE DISPOSABLE TYPE**
VERFAHREN ZUR HERSTELLUNG VON WINDELHÖSCHEN VOM EINWEGTYP
PROCEDE DE FABRICATION DE COUCHES-CULOTTES JETABLES

(30) Priority: 10.01.2003 SE 0300032
(43) Date of publication of application: 05.10.2005
(73) Proprietor: SCA Hygiene Products AB, 405 03 Göteborg (SE)
(72) Inventor: EEN, Hans, S-435 41 Mölnlycke (SE); HERMANSSON, Kent, S-426 54 Västra Frölunda (SE); SANDIN, Cécile, S-431 36 Mölndal (SE); STRANNEMALM, Kenneth, S-448 31 Floda (SE)
(74) Representative: Hyltner, Jan-Olof
(86) International application number: PCT/SE2004/000010
(87) International publication number: WO 2004/062541

(56) References cited:
- EP-A2- 0 187 728
- US-A- 6 042 673

## Description

### TECHNICAL FIELD

The present invention relates to a method using a blank for an absorbent article in the form of diaper pants or a sanitary panty in order to produce such an article, which blank includes an absorbent body enclosed between an inner, liquid-permeable cover sheet and an outer cover sheet, at least one cover sheet being made of a material which can be thermally welded, which blank further has a front portion, a rear portion, and a crotch portion which extends between these portions and includes and is delimited by the leg openings of the blank, said front portion and rear portion having side parts which extend laterally outside the absorbent body from the waist band to the leg openings. The invention also relates to an article of the disposable type produced by means of this method.

### PRIOR ART

Diaper pants are an alternative to open type diapers and have become increasingly popular for slightly older infants. Today's diaper pants have a good fit, which contributes to ensuring that they function well and which in addition gives them an aesthetically attractive look. Unlike conventional open diapers which look like pants once they have been fitted on the user, diaper pants look like pants right from the start, because the manufacturer has joined the side-edge portions of the front and rear portions of the diaper together. Diaper pants are easy to change with the user standing, by pulling them down just like underpants and then putting on a new set of diaper pants. US 3,604,015 discloses diaper pants in which a blank with front and rear portions of the same width is folded about a transverse line in the crotch portion, after which the edge portions of the folded blank which have been laid on one another are sewn together. Such a connection between the side-edge portions protrudes like a flange from the diaper pants and detracts from their appearance. To improve the appearance, these known diaper pants are produced by a method in which, in a final stage, the blank for the diaper pants is turned inside out so that said flange is situated on the inside of the diaper pants and does not therefore spoil the appearance of the diaper pants. A further disadvantage of such a flange connection is that it is exposed to forces (peel forces) directed at right angles to the connected surfaces and for this reason it must be very strong.

WO 99/44559 and US 5,607,537 disclose methods of producing flangeless seams and thereby avoiding peel forces. However, these seams comprise many material layers and are therefore bulky.

It is difficult to pull the diaper pants off when the user is lying down. If the diaper pants contain excrement, it is virtually impossible to pull the diaper pants off without soiling the user or his/her clothes. To solve this problem, it is known to design diaper pants such that they can be opened along the sides in the same way as conventional open diapers. For example, from EP-B1-0 755 238 it is known to connect the side-edge portions of the front and rear portions with the aid of detachable and resealable fastenings. From GB-A-2 267 024 it is known to arrange weakening lines in the side portions of the diaper pants in order to allow the diaper pants to be opened, and the diaper pants in EP-A2-0 187 728 have side seams which can be torn open. WO 88/07337 discloses diaper pants having welded side seams but does not describe how such seams are manufactured. US-A- 6,042,673 discloses a diaper pants having conductive members incorporated in the side parts in order to enable joining together the side parts by application of an electromagnetic field.

The present invention concerns in the first instance a method for producing diaper pants which ensures an aesthetically pleasing connection between the side-edge portions of the front and rear portions of the diaper pants without any step of turning the blank inside out, and which connection is designed such that the forces exerted on the diaper pants are largely taken up as shearing forces, and which method makes use of the fact that the cover sheets included in the diaper pants can be welded using heat. Secondly, the invention relates to a method by which it is possible to obtain a connection between the side-edge portions of the front and rear portions which reliably withstands the forces to which the diaper pants are exposed during normal use and handling, but which can nevertheless be easily torn open by an adult.

### DISCLOSURE OF THE INVENTION

These objects are achieved by means of a method using a blank for an absorbent article in the form of diaper pants or a sanitary panty in order to produce such an article, which blank includes an absorbent body enclosed between an inner, liquid-permeable cover sheet and an outer cover sheet, at least one cover sheet being made of a material which can be thermally welded, which blank further has a front portion, a rear portion, and a crotch portion which extends between these portions and includes and is delimited by the leg openings of the blank, said front portion and rear portion having side parts which extend laterally outside the absorbent body from the waist band to the leg openings, characterized by the following steps:
side-edge portions of the side parts of either the rear portion or front portion are folded in towards the inner cover sheet over supports which have been placed on the side parts of this portion and which extend along the full length of this portion, after which
the blank is folded about a transverse line in the crotch portion so that the end edges of the front portion and rear portion, forming the waist band of the finished article, come to lie edge-to-edge and so that the folded-in side edge portions of the side parts of either the rear portions or front portion overlap the side edge portions of the other portion, and thereafter
the overlapping side-edge portions of the side parts are welded together within the area of the supports by means of ultrasonic or thermal welding. Because the side-edge portions are connected overlapping one another, an aesthetically pleasing weld seam is obtained without turning the blank inside out, which weld seam is exposed only to shear forces and can thus be made to be tearable. Furthermore, the side-edge portions only overlap one another singly, which means that the portions joined together are not bulky.

According to a preferred embodiment, the supports are placed on the side parts before the side-edge portions of the side parts of the rear portion or front portion are folded in, and this folding is obtained by turning the supports. Furthermore, the front portion and rear portion of the blank are given the same width in the initial state and the side-edge portions of the side parts of the portion of the front portion and rear portion which are to have unfolded side-edge portions are offset sideways towards each other by a distance which corresponds to the reduction in width as a result of the folding-in of the side-edge portions of the second portion before folding the blank about a transverse line in the crotch portion. At least those parts of the side-edge portions of the side parts of the portion of front portion and rear portion which is to have unfolded side edge portions and extends within the areas of the supports are kept in a relaxed and unstressed state during the folding and welding steps.

Alternatively, the portion of front portion and rear portion which is to have unfolded side edge portions is given a width corresponding to the width of the second portion after its side-edge portions have been folded in across the supports.

The invention also relates to an absorbent article in the form of diaper pants including an absorbent body enclosed between an inner, liquid-permeable cover sheet and an outer cover sheet, at least one of said cover sheets being made of a material that can be thermally welded, which article further has a front portion, a rear portion, and a crotch portion which extends between these portions and which includes and is delimited by the leg openings of the blank, the front portion and rear portion having side parts which extend laterally outside the absorbent body from the waist opening to the leg openings and which are at least partially elastic, the side-edge portions of the front portion and of the rear portion overlap the side-edge portions of the second portion of these portions without comprising folded parts and are connected to these side-edge portions by means of a weld seam which extends along the full length of the side-edge portions. characterized in that the innermost of the overlapping side edge portions of the front or rear portion ends in a grip part situated outside said weld seam.

In a preferred embodiment, the outermost of the overlapping side edge portions has a length of less than 10 mm, preferably less than 7 mm, most preferably less than 4 mm. Preferably, the weld seam has a width of 6-10 mm, preferably 7-9 mm, and most preferably 8 mm, and it can be torn open. The weld seam preferably has a strength of 10-45 N/25mm.

### DESCRIPTION OF THE DRAWINGS

The invention will now be described with reference to the attached figures, of which:
Fig. 1 shows a diagrammatic perspective view of diaper pants according to one embodiment of the invention,
Fig. 2 shows a plan view illustrating diagrammatically a production line for producing diaper pants,
Figures 3-5 show diagrammatic cross sections of an edge portion of a blank for diaper pants during application of a weld support according to a first embodiment of the method according to the invention,
Fig. 6 shows a diagrammatic cross section of edge portions of the front and rear portions of the diaper pants blank which have been folded together before the welding operation has been performed,
Fig. 7 shows a view similar to Figure 6 after the welding operation has been performed,
Figures 8 and 9 show diagrammatic cross sections of an edge portion of a diaper pants blank during application of a weld support according to a second embodiment of the method according to the invention, and
Figures 10 and 11 illustrate a method of measuring the weld strength.

### DESCRIPTION OF EMBODIMENTS

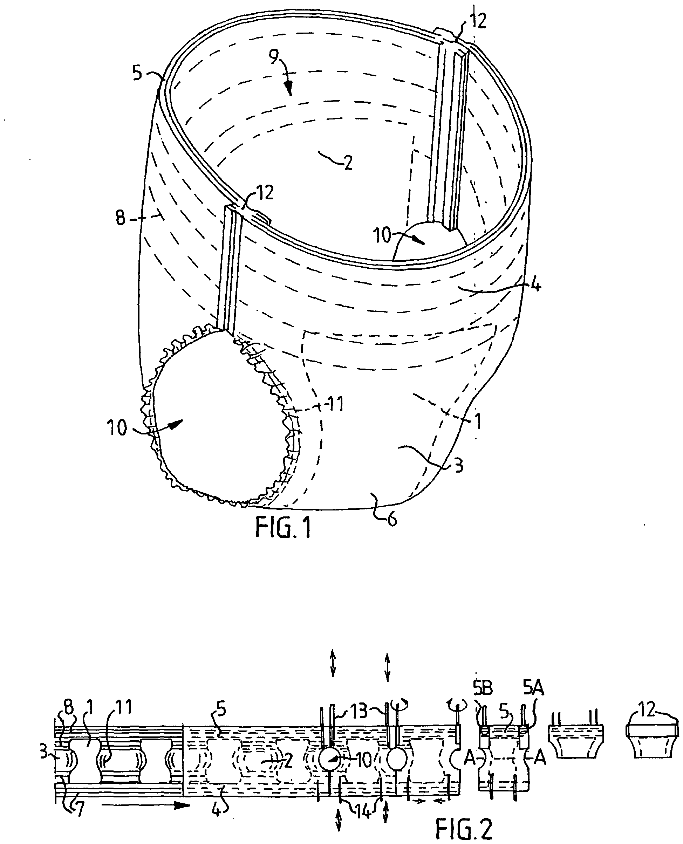

A preferred embodiment of diaper pants according to the invention is shown in Fig. 1. The diaper pants comprise an absorbent body 1 enclosed between an inner, liquid-permeable cover sheet 2 and an outer liquid-tight cover sheet 3. The cover sheets 2 and 3 are of identical shape and extend outside the absorbent body 1 around its entire circumference. In the parts lying outside the absorbent body 1, the cover sheets 2, 3 are connected to each other, for example by welding or gluing. The diaper pants have a front portion 4, a rear portion 5, and a crotch portion 6 lying between these portions.

The cover sheets are elastic, at least at the waist band and in the side panels of the front and rear portions 4, 5 lying laterally outside the absorbent body 1. In the embodiment shown, the elasticity of the cover sheets has been obtained by elastic threads 7, 8, which in a stretched state having been arranged between the cover sheets and secured to these by welding or gluing. The elasticity can also be obtained in other ways, for example by one of the cover sheets or both of them being made of elastic material. If both cover sheets are made of elastic material, one of the sheets does not need to be arranged in the stretched state, which is necessary if only one of the sheets is elastic. It is also possible for the side panels of the front and rear portions 4, 5 lying laterally outside the absorbent body 1 to consist of separate panels made of elastic material, while the waist band is made elastic by arranging stretched elastic threads or bands between the cover sheets 2, 3.

The diaper pants are shaped like pants with a waist opening 9 and two leg openings 10. Leg elastic in the form of stretched elastic threads 11 are arranged around the leg openings 10.

The mutually facing side-edge portions of the front and rear portions 4, 5 of the diaper pants overlap one another and are connected to one another by means of weld seams 12 which are generated by ultrasonic welding or thermal welding. The weld seams have a width of 6-10 mm, preferably 7-9 mm, and most preferably 8 mm, and a strength of 10-45 N/25mm measured in the manner set out below. Such a seam is able, with good margins, to cope with all normally occurring loads which arise during normal use of the diaper pants, but can still be easily torn open by an adult. In this way, the diaper pants can be removed from a user lying down, in the same way as an open diaper, which means that diaper pants containing excrement can be changed with minimal risk of soiling of the user's clothes.

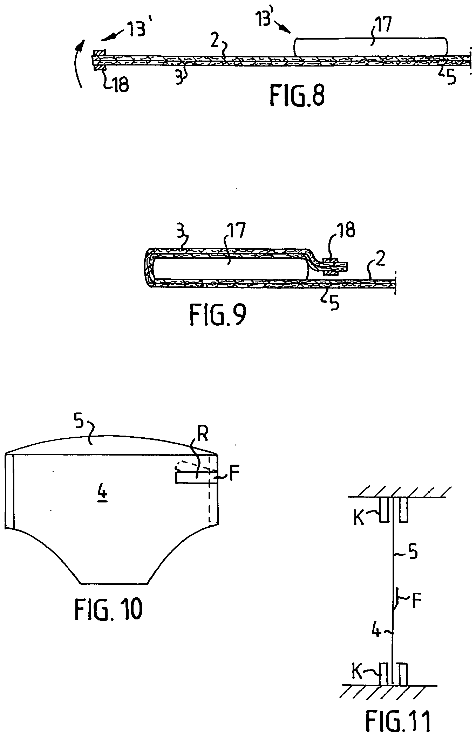

The seam strength is measured in the following way. First, in the manner illustrated in Figure 10, a strip R with a width of 25 mm is cut out from the front 4 and rear 5 side parts of diaper pants, which side parts are connected to one another by a seam F. The ends of this strip R are then clamped securely in the clamps K of a stress gauge, which clamps have a width of 25 mm. The clamps are then moved from one another at a speed of 300 mm/min and the force upon rupture of the seam or strip is recorded.

The liquid-permeable cover sheet 2 consists, for example, of a nonwoven, e.g. a spunbond nonwoven, a carded nonwoven, a meltblown nonwoven or a nonwoven laminate, e.g. a spunbond-meltblown-spunbond (SMS) laminate. The fibres used to build up the nonwoven material can consist of fibres of polyolefins, for example polyethylene or polypropylene, or of polyester. The nonwoven material can further consist of a mixture of several different types of fibres or of fibres which consist of several different polymers, called copolymers. Other materials which are used for liquid-permeable cover sheets, the so-called top sheets, for absorbent articles such as nonwovens of synthetic and/or natural fibres, perforated plastic sheets or laminates of these materials, can of course also be used as cover sheet 2.

The outer cover sheet 3 can consist of a nonwoven or a material built up from several nonwoven sheets, for example an SMS material. It can also consist of a plastic sheet, preferably of the breathable type, or a laminate of a plastic sheet and a nonwoven. All materials used as the so-called reverse sheet for absorbent articles can be used.

To obtain the weld seams 12, it is sufficient for one of the cover sheets 2, 3 to be made of material that can be thermally welded. However, it is preferable for both cover sheets to be made of materials that can be thermally welded, preferably also of materials whose melt points are similar to one another. For this reason, both the cover sheets 2, 3 are preferably made of material containing fibres which can be completely or partially melted.

The absorbent body 1 preferably comprises a sheet of cellulose fibres with or without admixture of superabsorbents and/or binder fibres. Other materials such as foamed material or moss can also be used separately or in combination with cellulose fibres. The absorbent body can also be built up from several sheets and advantageously comprises a sheet of material having a high permeability, for example a sheet of wadding. If the outer cover sheet 3 is not liquid-tight, the absorbent body comprises an impermeable sheet on its side facing the outer cover sheet.

If the side panels are made of elastic material, this can consist of elastomers produced from block copolymers, such as polyurethanes, copolyether esters, polyamide-polyether block copolymers, ethylene-vinylacetates (EVA) and the like, including polyurethanes available from E. I. Du Pont de Nemours Co., USA under the tradename LYCRA^{®} (also know as "spandex"); elastomeric styrene-butadiene copolymers, including those such as KRATON^{®} material available from Shell Chemical Company of Houston, Texas, USA; tetrablock copolymers including elastomeric styrene-poly(ethylene-propylene) block copolymers available from Shell Chemical Company of Houston, Texas, USA under the tradename KRATON^{®}; polyamides including polyether block amides available from Ato Chemical Company, USA under the tradename PEBAX^{®}; polyesters such as those available from E. I. Du Pont de Nemours Co. under the trade name HYTREL^{®}; single-site or metallocene-catalyzed polymers including single-site or metallocene-catalyzed polyolefins with a density of less than about 0.89 g/cm³ from Dow Chemical Co., USA under the tradename AFFINITY^{®}; and natural and synthetic rubber.

An embodiment of a method for producing diaper pants according to Figure 1 will now be described with reference to Figures 2-7. To simplify a comparison with the diaper pants in Figure 1, components in Figures 2-7 have been given the same reference numbers as the corresponding components in the finished diaper pants in Figure 1. For example, the web of liquid-permeable material in Figures 2-7 has been given the same reference number as the cover sheet 2 in the finished diaper pants.

In order to facilitate tearing open of the weld seam 12 a grip part is provided on the inside of diaper pant shown in Figure 1. By placing such a grip part on the inside of the diaper pant instead of on the outside no chafing against the arms of the wearer can occur, no tampering thereof by the child will occur and the diaper pant will have a better aesthetic appearance than if a loose free end edge part will be present on the outside. Such a grip part should in the circumferential direction preferably have a length of 10 mm or more. The free edge part on the outside of the diaper pant should have as small length in the circumferential direction as possible and should be less than 10 mm, preferably less than 7 mm and most preferably be less than 4 mm.

Diaper pants according to Figure 1 are produced in the following way.

Absorbent bodies 1 are laid on a running material web 3 of liquid-tight material, for example with the aid of a transfer wheel on which absorbent bodies 1 formed in a mat former wheel (not shown) have been placed. If the absorbent bodies 1 can be formed in synchrony with the advance of the material web 3, the transfer wheel can be omitted and the wheel can consist of a mat former wheel. The absorbent bodies 1 are deposited on the material web 3 in a row with the longitudinal direction of the bodies coinciding with the transverse direction of the web 3, i.e. at right angles to the direction of feed of the web 3 as is indicated by an arrow in Figure 2. In the embodiment shown, the diaper pants are thus produced in what is called crosswise production. Before the absorbent bodies 1 have been deposited on the web 3, elastic threads 7, 8 and 11, forming the waist band, elastic side panels and leg elastic of the produced diaper pants, have been applied in the stretched state.

A material web 2 of liquid-permeable material is then placed on top of the row of absorbent bodies 1. The material web 2 can pass through a gluing unit immediately before application and is secured, with the aid of a pair of rollers, to the material web 3 in parts lying outside the absorbent bodies 1. If appropriate, the material web 2 is also secured to the rear side of each absorbent body 1. The elastic threads are expediently secured at the same time to the cover sheets, although it is of course possible to secure these to the material web 3 before the absorbent bodies have been applied. Instead of gluing, it is also possible to use ultrasonic or thermal welding to secure the cover sheets to each other and to secure the elastic threads or bands to one or both of the cover sheets 2, 3. A web of continuous diaper pants blanks is obtained by means of both cover sheets having been connected to one another. It should be noted that the production steps described thus far are conventional and well known to the skilled person.

Gripping and support members 13 are thereafter applied to the area which is to constitute the side-edge portions of the rear portion 5 of finished diaper pants, and gripping members 14 are arranged on the area which is to constitute the side-edge portions of the front portion 4 at a distance from the respective side edge of between 10 and 40 mm, preferably between 15 and 35 mm, and most preferably between 20 and 25 mm. Individual diaper pants blanks with leg openings 10 are then cut out from the web of continuous diaper pants blanks. The leg openings can of course be cut out before application of the gripping and support members 13.

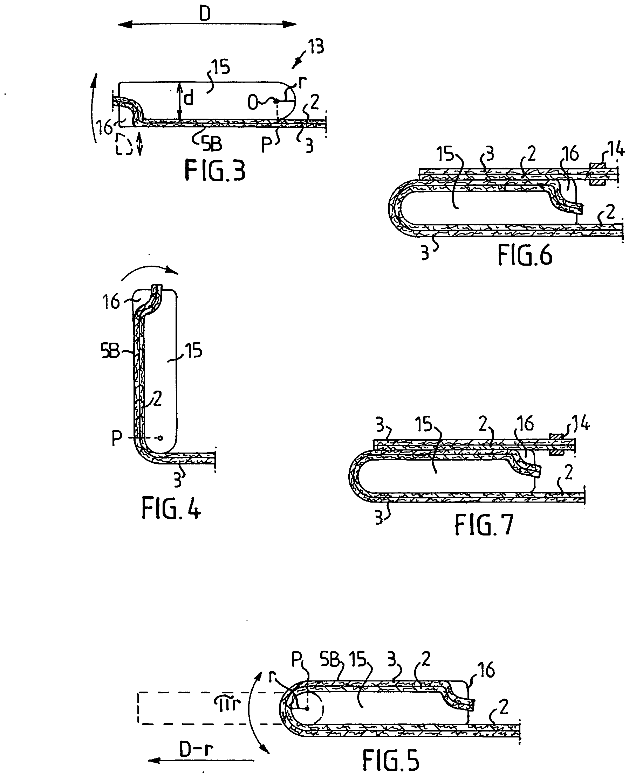

The side-edge portions 5A, 5B of the rear portion 5 of the diaper pants blank are then folded in across the inner liquid-permeable cover sheet 2 with the aid of the gripping and support members 13 so that the outer cover sheet 3 is facing the observer of Figure 2 in these edge portions, and so that a support part 14 of the gripping and support member comes to lie between the folded-in part of the side-edge portion and the underlying part of the rear portion 5. This folding operation is described in more detail with reference to Figures 3-5. At the same time, the gripping members 14 are displaced symmetrically, in relation to the longitudinal axis of symmetry of the diaper pants blank, towards each other in the transverse direction of the diaper pants blank by a distance which corresponds to twice the width of the folded-in part of the side-edge portion of the rear portion.

Figures 3-5 show the folding-in of the side-edge portion 5B of the rear portion 5 in greater detail. As can be seen from Figure 3, the gripping and support member 13 is made up of two main parts, namely a support part 15 and a clamp part 16. The clamp part 16 is movable from the position shown by a broken line in Figure 3 to come to bear against a recess in the support 15. The member 13 has a width D of 15-40 mm and a thickness d of 5-25 mm. In the embodiment shown, the members 13 are displaceable transversely with respect to the web of continuous diaper pants blanks, as is indicated by arrows in Figure 2, and can also move in the direction of advance of the web. However, it is conceivable for the members 13 to be displaceable only in the direction of feed of the web. In such a case, however, one of the parts 15, 16 must be able to be pivoted at right angles to the other part and to the plane shown in Figure 2 to ensure that the members 13 can be applied to the web of continuous diaper pants blanks. After the members 13 have been applied to the web of continuous diaper pants blanks, the cover sheets are clamped securely between the parts 15 and 16. After the continuous web of diaper pants blanks has been separated in the transverse direction, the member 13 can begin to be turned about an axis O. In Figure 4, the member 13 is shown when it has been turned through 90°, and in Figure 5 when it has been turned through 180°. In Figure 5, the broken lines show the position of the member 13 before turning. As can be seen from Figure 5, the effect of the turning movement is that the outer edge of that side part of which the portion 5B forms a part has been displaced by the distance D-r in the direction towards the longitudinal axis of symmetry of the diaper pants blanks in relation to the position of this outer edge in the unfolded state of the side-edge portion 5B. r represents the distance between the rotation axis O and the nearest longitudinal edge of the member 13. In the embodiment shown, this edge has a semicircular shape. In Figures 3-5, a point P is shown at which the cover sheets 2, 3 are in contact with the member 13 both in the unfolded and in the folded state. During rotation of the member 13, this point P is displaced along the distance πr, i.e. the arc length of the semicircular edge. This means that if the member 13 is stationary during the rotation movement, the cover sheets 2, 3 have to be stretched by a distance which corresponds to the arc length πr during the rotation movement of this member. This does not pose a problem if the side panels are made of elastic material, but in the present case when the elastic threads have been applied in the stretched state onto unfolded material webs, the member 13 has to be moved sideways towards the longitudinal axis of symmetry of the diaper pants blank by a distance corresponding to the arc length before or during the rotation movement of the member 13. This distance must be added to the abovementioned displacement of the outer edge in order to obtain the distance by which the outer edge has approached the longitudinal axis of symmetry of the diaper pants blank. Rotation of the opposite side-edge portion 5A gives rise to a corresponding displacement of the outer edge of the opposite side part. The distance by which the gripping members 14 for the side-edge portions of the front portion are displaced towards one another corresponds expediently to the last-mentioned distance minus the shortening of these edge portions' outermost parts caused by the elastic threads 7 being allowed to contract from the stretched state to the relaxed state in those parts lying outside the gripping members 14. Relaxed state is intended to signify the state which these elastic threads assume when the diaper pants or the diaper pants blank are not subjected to external stresses.

The longitudinal edge of the member 13 lying nearest the rotation axis O can of course have a cross-sectional shape other than semicircular and thus have an arc length different than that shown in the embodiment.

When the displacements of the members 13, 14 and the rotation of the members 13 have been executed, the diaper pants blank is folded about a transverse line A-A in the crotch portion so that the end edges of the front and rear portions 4, 5, which form the waist band of the finished diaper pants, come to lie edge-to-edge and so that the parts of the side-edge portions of the front portion 4 lying outside the grip members 14 come to bear against the folded-in parts of the side-edge portions of the rear portion. Throughout the folding operation, the side-edge portions are held securely by the members 13, 14 which are thus entrained with the folding of the diaper pants blank. Figure 6 shows a cross-sectional view of the edge portions of the front and rear portions 4, 5 of the diaper pants blank after the front portion 4 has been folded in across the rear portion 5. As can be seen from this figure, the gripping members 14 are situated inside the gripping and support members 13 as viewed in the sideways direction, which means that the side-edge portions are brought to bear against one another through the folding operation. The members 13, 14 are expediently arranged on a foldable frame which is either designed so that one half is foldable towards the other half, or so that the halves are folded against each other. In the latter case, the diaper pants blank, after folding, comes to lie in a plane at right angles to the plane in Figure 2.

The folded-together diaper pants blank is then moved past ultrasonic horns or thermal welding jaws which generate weld seams 12. Figure 7 shows the diaper pants directly after the side-edge portions have been welded together.

The supports 15, which are preferably made of metal, for example iron, ensure that only the folded-in part of the side-edge portions of the rear portion 5 are secured to the side-edge portion of the front portion 4. The weld seams can consist of continuous seams or of patterns of spot welds or line welds.

After the welding has been carried out, the members 13, 14 are withdrawn from the finished diaper pants either by moving the members upwards in Figure 2 or by pulling the diaper pants downwards from these members in Figure 2. The diaper pants are then conveyed to a packaging station.

In the embodiment of the method according to the invention shown in Figure 2, the welding is carried out intermittently, i.e. the diaper pants blank is held stationary during the welding operation. If instead the production line is to be completely continuous, the diaper pants blanks are preferably conveyed past stationary ultrasonic horns during displacement in the longitudinal direction. This can be done either by rotating the diaper pants blanks and the members gripping these, or by changing the direction of feed (change of conveyor belt). Another way is to use ultrasonic horns which are displaceable both in the direction of feed and in the transverse direction.

In the embodiment described, the web 2 of liquid-permeable material is placed on top of the web 3 of outer cover sheet material after absorbent bodies have been deposited thereon. It is of course possible for the webs to be changed around so that absorbent bodies are deposited on the web of liquid-permeable material, and the web of outer cover sheet material is applied last. The gripping and support members 13, 14 are in this case arranged on the underside of the web of diaper pants blanks, and the members 13 are rotated in opposite directions from those illustrated in Figure 2.

Figures 8 and 9 show an alternative embodiment of a gripping and support member 13'. This member differs from the member 13 in that the support part 17 is not rotatable and the gripping member 18 does not consist of a part integrated in the support part, but instead of a pivotable clamping fork 18. The folding-in of the side-edge portion of the rear portion 5 across the support 17 takes place exclusively by pivoting of the clamping fork 18 to the position shown in Figure 9. It should be noted that the distance between the support part 17 and the clamping fork 18 is chosen so that the clamping fork 18 can be brought to bear against the cover sheet 2 of the rear portion 5, as is indicated in Figure 9. This means that the side-edge portion of the front portion 4 can be brought to bear against the folded-in part of the rear portion 5 upon folding of the diaper pants blank about a transverse line in the crotch portion.

In both of the embodiment shown in figures 3-9 the parts gripped by gripping members 15,16 and 18, respectively will constitute grip parts in the produced diaper pants. From Figure 6 it is also evident that it is possible to weld the side part of the front portion to the portion 5B of the rear part so that no free edge of the side part of the front portion will be present in the produced diaper pants.

The described diaper pants can be designed for young children and also for incontinent adults.

The described embodiments can of course be modified within the scope of the invention. For example, the method can also be applied on production lines with so-called lengthways production, i.e. the diaper pants blanks have their longitudinal direction coincident with the direction of feed of the material webs, and the absorbent bodies can have another shape than the hourglass shape shown in the figures, for example rectangular. The supports 15 and 17 can be made of other materials, for example ceramic material, and the gripping members can be designed in a way other than that described. In the described embodiments, the front and rear portions of the diaper pants blanks are identical, but this is not necessary. For example, the side parts of the front or rear portion which are not to be folded could be given a smaller width than the side parts of the second portion. In such a configuration, the gripping members 14 do not have to be moved towards one another in order to ensure that the side-edge portions which are to be connected to each other will overlap after the diaper pants blank has been folded about a transverse fold line. To avoid waste, such a configuration should be used in crosswise production, the front portions of the diaper pants blanks being alternately located on different sides. The invention is therefore limited only by the content of the attached patent claims.

## Claims

1. Method using a blank for an absorbent article in the form of diaper pants in order to produce such an article, which blank includes an absorbent body (1) enclosed between an inner, liquid-permeable cover sheet (2) and an outer cover sheet (3), at least one cover sheet being made of a material which can be thermally welded, which blank further has a front portion (4), a rear portion (5), and a crotch portion (6) which extends between these portions and includes and is delimited by the leg openings (10) of the blank, said front portion and rear portion having side parts which extend laterally outside the absorbent body from the waist band to the leg openings (10), **characterized by** the following steps:
side-edge portions (5A, 5B) of the side parts of either the rear portion (5) or front portion (4) are folded in towards the inner cover sheet (2) over supports (15; 17) which have been placed in the side parts of this portion and which extend along the full length of this portion, after which
the blank is folded about a transverse line in the crotch portion (6) so that the end edges of the front portion (4) and rear portion (5) come to lie edge-to-edge and so that the folded-in side edge portions of the side parts of either the rear portions (4) or front portion (4) overlap the side edge portions of the other portion (5), and thereafter the overlapping side-edge portions of the side parts are welded together within the area of the supports (15; 17) by means of ultrasonic or thermal welding.

2. Method according to Claim 1, **characterized in that** the supports (15) are placed on the side parts before the side-edge portions of the side parts of the rear portion or front portion are folded in, and **in that** this folding is obtained by turning the supports (15).

3. Method according to Claim 1 or 2, **characterized in that** the front portion (4) and rear portion (5) of the blank are given the same width, and **in that** the side-edge portions of the side parts of the portion (4) of front portion and rear portion which is to have unfolded side edge portions are offset sideways towards each other by a distance which corresponds to the reduction in width as a result of the folding-in of the side-edge portions of the second portion (5) before folding the blank about a transverse line in the crotch portion (6).

4. Method according to Claim 3, **characterized in that** at least those parts, extending within the areas for the supports (15; 17), of the side-edge portions of the side parts of the portion (4) of front portion (4) and rear portion (5) which is to have unfolded side-edge portions are kept in a relaxed and unstressed state during the folding and welding steps.

5. Method according to Claim 1 or 2, **characterized in that** the portion of front portion and rear portion which is to have unfolded side edge portions is given a width corresponding to the width of the second portion after its side-edge portions have been folded in over the supports.

6. Absorbent article in the form of diaper pants or a sanitary panty with a waist opening (9) and two leg openings (10), which article includes an absorbent body (1) enclosed between an inner, liquid-permeable cover sheet (2) and an outer cover sheet (3), at least one of said cover sheets being made of a material that can be thermally welded, which article further has a front portion (4), a rear portion (5), and a crotch portion (6) which extends between these portions and which includes and is delimited by the leg openings (10) of the blank, the front portion and rear portion having side parts which extend laterally outside the absorbent body from the waist opening (9) to the leg openings (10) and which are at least partially elastic, the side-edge portions of the front portion (4) or rear portion (5) overlap the side-edge portions of the second portion of these portions without comprising folded parts and are connected to the second side-edge portions by means of a weld seam (12) which extends along the full length of the side-edge portions. **characterized in that** the innermost of the overlapping side edge portions of the front or rear portion ends in a grip part situated outside said weld seam (12).

7. Absorbent article according to Claim 6, **characterized in that** the outermost of the overlapping side edge portions has a length of less than 10 mm, preferably less than 7 mm, most preferably less than 4 mm.

8. Absorbent article according to Claim 6 or 7, **characterized in that** the weld seam (12) has a width of 6-10 mm, preferably 7-9 mm, and most preferably 8 mm.

9. Absorbent article according to Claim 8, **characterized in that** the weld seam (12) can be torn open.

10. Absorbent article according to Claim 9, **characterized in that** the weld seam has a strength of 10-45 N/25mm.

## Patentansprüche

1. Verfahren, bei welchem ein Rohling für einen absorbierenden Artikel in Form von Windelhöschen zur Herstellung eines solchen Artikels verwendet wird, welcher Rohling einen zwischen einem inneren, flüssigkeitsdurchlässigen Deckblatt (2) und einem äußeren Deckblatt (3) eingebetteten absorbierenden Körper (1) umfasst, wobei mindestens ein Deckblatt aus einem thermisch verschweißbaren Werkstoff hergestellt ist, welcher Rohling ferner einen Vorderteil (4), einen Hinterteil (5) und einen sich zwischen diesen Teilen erstreckenden Schrittteil (6) aufweist, welcher die Beinöffnungen (10) des Rohlings umfasst und durch diese abgegrenzt ist, wobei der Vorderteil und der Hinterteil Seitenteile umfassen, welche sich außerhalb des absorbierenden Körpers vom Bund bis zu den Beinöffnungen (10) seitlich erstrecken, **gekennzeichnet durch** die folgenden Schritte:
Einfalten von Seitenrandteilen (5A, 5B) der Seitenteile von entweder dem Hinterteil (5) oder dem Vorderteil (4) gegen das innere Deckblatt (2) über in den Seitenteilen dieses Teils angeordnete, sich entlang der ganzen Länge dieses Teils erstreckende Unterstützungen (15; 17), und nachträglich Falten des Rohlings um eine Querlinie im Schrittteil (6), so dass die Endränder des Vorderteils (4) und des Hinterteils (5) von Rand zu Rand zu liegen kommen, und so dass die eingefalteten Seitenrandteile der Seitenteile von entweder dem Hinterteil (4) oder dem Vorderteil (4) die Seitenrandteile des anderen Teils (5) überlappen, und danach
Zusammenschweißen der überlappenden Seitenrandteile der Seitenteile mit dem Bereich der Unterstützungen (15; 17) **durch** Ultraschallschweißen oder thermisches Schweißen.

2. Verfahren nach Anspruch 1, **dadurch gekennzeichnet, dass** die Unterstützungen (15) vor dem Einfalten der Seitenrandteile der Seitenteile des Hinterteils oder des Vorderteils an den Seitenteilen angeordnet werden, und dass dieses Einfalten durch ein Drehen der Unterstützungen (15) erreicht wird.

3. Verfahren nach Anspruch 1 oder 2, **dadurch gekennzeichnet, dass** der Vorderteil (4) und der Hinterteil (5) des Rohlings dieselbe Breite erhalten, und dass die Seitenrandteile der Seitenteile des Teils (4) des Vorderteils und des Hinterteils, deren Seitenrandteile entfaltet sein müssen, um eine Strecke seitlich gegeneinander versetzt werden, welche der Reduktion der Breite als Ergebnis des Einfaltens der Seitenrandteile des zweiten Teils (5) vor dem Einfalten des Rohlings um eine Querlinie im Schrittteil (6) entspricht.

4. Verfahren nach Anspruch 3, **dadurch gekennzeichnet, dass** mindestens die sich innerhalb der Bereiche für die Unterstützungen (15; 17) erstreckenden Teile der Seitenrandteile der Seitenteile des Teils (4) des Vorderteils (4) und des Hinterteils (5), deren Seitenrandteile entfaltet sein müssen, während der Schritte des Einfaltens und Verschweißens in einem entspannten und unbeanspruchten Zustand gehalten werden.

5. Verfahren nach Anspruch 1 oder 2, **dadurch gekennzeichnet, dass** der Teil des Vorderteils und des Hinterteils, deren Seitenrandteile entfaltet sein müssen, eine Breite erhalten, welche der Breite des zweiten Teils nach dem Einfalten seiner Seitenrandteile über die Unterstützungen entspricht.

6. Absorbierender Artikel in For von Windelhöschen oder einer Hygienebinde mit Taillenöffnung (9) und zwei Beinöffnungen (10), welcher Artikel einen zwischen einem inneren, flüssigkeitsdurchlässigen Deckblatt (2) und einem äußeren Deckblatt (3) eingebetteten absorbierenden Körper (1) umfasst, wobei mindestens eines der Deckblätter aus einem thermisch verschweißbaren Werkstoff hergestellt ist, welcher Artikel ferner einen Vorderteil (4), einen Hinterteil (5) und einen sich zwischen diesen Teilen erstreckenden Schrittteil (6) aufweist, welcher die Beinöffnungen (10) des Rohlings umfasst und durch diese abgegrenzt ist, wobei der Vorderteil und der Hinterteil Seitenteile umfassen, welche sich außerhalb des absorbierenden Körpers von der Taillenöffnung (9) bis zu den Beinöffnungen (10) seitlich erstrecken und zumindest teilweise elastisch sind, wobei die Seitenrandteile des Vorderteils (4) oder des Hinterteils (5) die Seitenrandteile des zweiten Teils dieser Teile überlappen, ohne gefaltete Teile zu umfassen, und mittels einer sich entlang der ganzen Länge der Seitenrandteile erstreckenden Schweißnaht (12) mit den zweiten Seitenrandteilen verbunden sind, **dadurch gekennzeichnet, dass** das Innerste der überlappenden Seitenrandteile des Vorderteils oder des Hinterteils in einem außerhalb der Schweißnaht (12) angeordneten Greifteil endet.

7. Absorbierender Artikel nach Anspruch 6, **dadurch gekennzeichnet, dass** das Äußerste der überlappenden Seitenrandteile eine Länge von weniger als 10 mm, bevorzugt weniger als 7 mm, besonders bevorzugt weniger als 4 mm aufweist.

8. Absorbierender Artikel nach Anspruch 6 oder 7, **dadurch gekennzeichnet, dass** die Schweißnaht (12) eine Breite von 6-10 mm, bevorzugt von 7-9 mm und besonders bevorzugt von 8 mm aufweist.

9. Absorbierender Artikel nach Anspruch 8, **dadurch gekennzeichnet, dass** die Schweißnaht (12) aufgerissen werden kann.

10. Absorbierender Artikel nach Anspruch 9, **dadurch gekennzeichnet, dass** die Schweißnaht eine Stärke von 10-45 N/25 mm aufweist.

## Revendications

1. Procédé utilisant une ébauche pour un article absorbant dans la forme de couches-culottes afin de produire un tel article, ébauche qui comprend un corps absorbant (1) enserré entre une feuille de couverture intérieure (2) perméable aux liquides et une feuille de couverture extérieure (3), au moins une feuille de couverture étant faite d'un matériau qui peut être soudé thermiquement, ébauche qui présente en outre une partie avant (4), une partie arrière (5) et une partie d'entrejambe (6) qui s'étend entre ces parties et comprend et est délimitée par les ouvertures pour jambes (10) de l'ébauche, ladite partie avant et ladite partie arrière comportant des portions latérales qui s'étendent latéralement à l'extérieur du corps absorbant depuis la bande de taille jusqu'aux ouvertures pour jambes (10), **caractérisé par** les étapes suivantes:
des parties de bord latéral (5A, 5B) des portions latérales soit de la partie arrière (5) soit de la partie avant (4) sont repliées vers l'intérieur en direction de la feuille de couverture intérieure (2) au-dessus de supports (15; 17) qui ont été placés dans les portions latérales de cette partie et qui s'étendent sur toute la longueur de cette partie, ensuite
l'ébauche est repliée autour d'une ligne transversale dans la partie d'entrejambe (6) si bien que les bords d'extrémité de la partie avant (4) et de la partie arrière (5) viennent se placer bord à bord et si bien que les parties de bord latéral repliées vers l'intérieur des portions latérales soit des parties arrières (4) ou de la partie avant (4) chevauchent les parties de bord latéral de l'autre partie (5), et ensuite
les parties de bord latéral chevauchantes des portions latérales sont soudées ensemble au sein de la zone des supports (15; 17) au moyen de soudage ultrasonique ou thermique.

2. Procédé selon la revendication 1, **caractérisé en ce que** les supports (15) sont arrangés sur les portions latérales avant que les parties de bord latéral des portions latérales de la partie arrière ou de la partie avant ne soient repliées vers l'intérieur, et **en ce que** ce pliage est obtenu en tournant les supports (15).

3. Procédé selon la revendication 1 ou 2, **caractérisé en ce que** la partie avant (4) et la partie arrière (5) de l'ébauche sont pourvues de la même largeur, et **en ce que** les parties de bord latéral des portions latérales de la partie (4) de la partie avant et de la partie arrière qui est à avoir des parties de bord latéral dépliées sont décalées latéralement l'une vers l'autre par une distance qui correspond à la réduction de la largeur à la suite du pliage vers l'intérieur des parties de bord latéral de la deuxième partie (5) avant de plier l'ébauche autour d'une ligne transversale dans la partie d'entrejambe (6).

4. Procédé selon la revendication 3, **caractérisé en ce qu'**au moins ces parties, s'étendant dans les zones pour les supports (15; 17), des parties de bord latéral des portions latérales de la partie (4) de la partie avant (4) et de la partie arrière (5) qui est à avoir des parties de bord latéral dépliées, sont maintenues dans un état détendu et sans contrainte lors les étapes de pliage et de soudure.

5. Procédé selon la revendication 1 ou 2, **caractérisé en ce que** la partie de la partie avant et de la partie arrière qui est à avoir des parties de bord latéral dépliées est pourvue d'une largeur correspondant à la largeur de la deuxième partie, après que ses parties de bord latéral ont été pliées vers l'intérieur au-dessus des supports.

6. Article absorbant dans la forme de couches-culottes ou d'un slip sanitaire avec une ouverture de taille (9) et deux ouvertures pour jambes (10), article qui comprend un corps absorbant (1) enserré entre une feuille de couverture intérieure (2) perméable aux liquides et une feuille de couverture extérieure (3), au moins l'une desdites feuilles de couverture étant faite d'un matériau qui peut être soudé thermiquement, article qui présente en outre une partie avant (4), une partie arrière (5) et une partie d'entrejambe (6) qui s'étend entre ces parties et comprend et est délimitée par les ouvertures pour jambes (10) de l'ébauche, la partie avant et la partie arrière comportant des portions latérales qui s'étendent latéralement à l'extérieur du corps absorbant depuis l'ouverture de taille (9) jusqu'aux ouvertures pour jambes (10), et qui sont au moins partiellement élastiques, les parties de bord latéral de la partie avant (4) ou de la partie arrière (5) chevauchent les parties de bord latéral de la deuxième partie de ces parties sans comprendre des parties pliées et sont reliées aux deuxièmes parties de bord laréral au moyen d'un cordon de soudure (12) qui s'étend le long de la longueur totale des parties de bord latéral, **caractérisé en ce que** le coeur des parties de bord latéral chevauchantes de la partie avant ou de la partie arrière se termine dans une partie poignée située à l'extérieur dudit cordon de soudure (12).

7. Article absorbant selon la revendication 6, **caractérisé en ce que** les régions ultrapériphériques des parties de bord latéral chevauchantes présentent une longueur inférieure à 10 mm, de préférence inférieure à 7 mm, et le plus préférablement inférieure à 4 mm.

8. Article absorbant selon la revendication 6 ou 7, **caractérisé en ce que** le cordon de soudure (12) présente une largeur de 6 à 10 mm, de préférence de 7 à 9 mm, et le plus préférablement de 8 mm.

9. Article absorbant selon la revendication 8, **caractérisé en ce que** le cordon de soudure (12) peut être ouvert par déchirement.

10. Article absorbant selon la revendication 9, **caractérisé en ce que** le cordon de soudure présente une force de 10-45 /25mm.
